# EUROPEAN PATENT APPLICATION

(11) **EP 3 733 852 A1**
(43) Date of publication of application: **04.11.2020**
(21) Application number: 18895220.4
(22) Date of filing: 28.12.2018
(51) Int. Cl.: C12N 15/115

(54) **APTAMER TEMPLATE AND METHOD FOR PREPARING APTAMER BY USING SAME**

(30) Priority: 28.12.2017 KR 20170183058; 28.12.2017 KR 20170183057; 21.09.2018 KR 20180114416; 21.09.2018 KR 20180114417
(71) Applicant: NUCLIXBIO, Guro-gu Seoul 08377 (KR)
(72) Inventor: KANG, Hoyoung, Seoul 06624 (KR); LEE, Sunghak, Gwangmyeong-si, Gyeonggi-do 14241 (KR); KIM, Dohyung, Sejong 30100 (KR); YOUK, Yewon, Incheon 21521 (KR)
(74) Representative: Maiwald Patent- und Rechtsanwaltsgesellschaft mbH
(86) International application number: PCT/KR2018/016878
(87) International publication number: WO 2019/132597

(57) **Abstract**

Provided are a novel nucleic acid structure, its use as an aptamer template, and a method for preparing an aptamer using thereof.

<110>	Nuclixbio

<120>	Aptamer Template and Method of Preparing Aptamer Using The Same

<130>	OPP20185170KR

<150>	KR 10-2017-0183057
<151>	2017-12-28

<150>	KR 10-2017-0183058
<151>	2017-12-28

<150>	KR 10-2018-0114416
<151>	2018-09-21

<150>	KR 10-2018-0114417
<151>	2018-09-21

<160>	47

<170>	KopatentIn 3.0

<210>	1
<211>	40
<212>	DNA
<213>	Artificial Sequence

<220>
<223>	Synthetic (HFL aptamer)


<400>	1
ataggacgga tgagcgtccg gccggatggg ttttccatca	40


<210>	2
<211>	40
<212>	DNA
<213>	Artificial Sequence

<220>
<223>	Synthetic (HFL aptamer)


<400>	2
ctgggacgcg gatgagtccg ctccaccagc catccatgta	40


<210>	3
<211>	40
<212>	DNA
<213>	Artificial Sequence

<220>
<223>	Synthetic (HFL aptamer)


<400>	3
ttaggacgcg aagatgtcct tgcaagggct catcgcatca	40


<210>	4
<211>	40

<212>    DNA
<213>    Artificial Sequence

<220>
<223>    Synthetic (HFL aptamer)


<400>    4
atgggacgac cctgtgtccc tatatgcgcg gagagccatc                              40


<210>    5
<211>    40
<212>    DNA
<213>    Artificial Sequence

<220>
<223>    Synthetic (HFL aptamer)


<400>    5
cttggacgcc ctcgtgtccc ttgctccttc acatacgcaa                              40


<210>    6
<211>    40
<212>    DNA
<213>    Artificial Sequence

<220>
<223>    Synthetic (HFL aptamer)


<400>    6
gctggacgtt cgtgggtccc attctgaagc ctaagactgg                              40


<210>    7
<211>    40
<212>    DNA
<213>    Artificial Sequence

<220>
<223>    Synthetic (HFL aptamer)


<400>    7
gagggacgaa caagcgtcca gcggttatgc accttcggta                              40


<210>    8
<211>    40
<212>    DNA
<213>    Artificial Sequence

<220>
<223>    Synthetic (HFL aptamer)


<400>    8
tacggacgca cctccgtccg gttctcagcg tggggcgcac                              40

<210>	9
<211>	40
<212>	DNA
<213>	Artificial Sequence

<220>
<223>	Synthetic (HFL aptamer)


<400>	9
gcaggacgta ctgatgtccc gttgcatgtc gctaagctcc	40


<210>	10
<211>	40
<212>	DNA
<213>	Artificial Sequence

<220>
<223>	Synthetic (HFL aptamer)


<400>	10
cgcggacggg gttttgtccc ccgtagctga ctaaatacat	40


<210>	11
<211>	40
<212>	DNA
<213>	Artificial Sequence

<220>
<223>	Synthetic (HFL aptamer)


<400>	11
acgggacgcg ttctggtccc tacttcttgt gtgtctattc	40


<210>	12
<211>	40
<212>	DNA
<213>	Artificial Sequence

<220>
<223>	Synthetic (HFL aptamer)


<400>	12
aatggacgca tcccagtccc ccaagcttcc gtatcgtaac	40


<210>	13
<211>	40
<212>	DNA
<213>	Artificial Sequence

<220>
<223>	Synthetic (HFL aptamer)


<400>	13
tgcggacgca cgcctgtcct caacccatga ttacacgttt	40

<210>    14
<211>    40
<212>    DNA
<213>    Artificial Sequence

<220>
<223>    Synthetic (HFL aptamer)


<400>    14
tggggacggg tatacgtcca tctcgtatcc aactaggcga                              40


<210>    15
<211>    40
<212>    DNA
<213>    Artificial Sequence

<220>
<223>    Synthetic (HFL aptamer)


<400>    15
gtgggacgcg tgtaggtcca aacggtgcgg tttggcgctc                              40


<210>    16
<211>    40
<212>    DNA
<213>    Artificial Sequence

<220>
<223>    Synthetic (HFL aptamer)


<400>    16
ggaggacgag tggtcgtccc gctgggcaat agctcggggc                              40


<210>    17
<211>    40
<212>    DNA
<213>    Artificial Sequence

<220>
<223>    Synthetic (HFL aptamer)


<400>    17
acaggacgcc agagcgtcca aggtcgccgc tagtagaccc                              40


<210>    18
<211>    40
<212>    DNA
<213>    Artificial Sequence

<220>
<223>    Synthetic (HFL aptamer)

<400>    18
tccggacgta gcccggtccc agcgcacggt accaccgtcg                              40


<210>    19
<211>    40
<212>    DNA
<213>    Artificial Sequence

<220>
<223>    Synthetic (HFL aptamer)


<400>    19
ccaggacgac tctaggtcct catgagatta gatatcggtt                              40


<210>    20
<211>    40
<212>    DNA
<213>    Artificial Sequence

<220>
<223>    Synthetic (HFL aptamer)


<400>    20
gtcggacgga ataacgtcct aaatatttat gcaacgggga                              40


<210>    21
<211>    40
<212>    DNA
<213>    Artificial Sequence

<220>
<223>    Synthetic (Random aptamer)


<400>    21
cctcttgcgc gctccgtatc acatttatat gatggccagt                              40


<210>    22
<211>    40
<212>    DNA
<213>    Artificial Sequence

<220>
<223>    Synthetic (Random aptamer)


<400>    22
tctcttacaa cgggtcttgg ccataacaca ataatcctgc                              40


<210>    23
<211>    40
<212>    DNA
<213>    Artificial Sequence

<220>
<223>    Synthetic (Random aptamer)

<400>    23
cctaactcga ccagtgggct cggacggatg attaacccta                              40


<210>    24
<211>    40
<212>    DNA
<213>    Artificial Sequence

<220>
<223>    Synthetic (Random aptamer)


<400>    24
cccctacatc cctttttgcc gcaaatcacc cagctcctga                              40


<210>    25
<211>    40
<212>    DNA
<213>    Artificial Sequence

<220>
<223>    Synthetic (Random aptamer)


<400>    25
cttccttgat cgctgcccga tttttggaat aggttcctag                              40


<210>    26
<211>    40
<212>    DNA
<213>    Artificial Sequence

<220>
<223>    Synthetic (Random aptamer)


<400>    26
ctcaggttgt cgatctaacc attggcgggt acgttgtacc                              40


<210>    27
<211>    40
<212>    DNA
<213>    Artificial Sequence

<220>
<223>    Synthetic (Random aptamer)


<400>    27
cccatgtcag ccggttaatc ttaatgttga ttcgagtcac                              40


<210>    28
<211>    40
<212>    DNA
<213>    Artificial Sequence

<220>
<223>    Synthetic (Random aptamer)


<400>    28
ctcttgtcgc agtacctcaa ttgagcgttg tacacattgt                              40


<210>    29
<211>    40
<212>    DNA
<213>    Artificial Sequence

<220>
<223>    Synthetic (Random aptamer)


<400>    29
tgtatttcag taatccaacg gcacttgtga cactgctact                              40


<210>    30
<211>    40
<212>    DNA
<213>    Artificial Sequence

<220>
<223>    Synthetic (Random aptamer)


<400>    30
tactcagacg aatcaaatat cgtgtccaaa gtcctccgag                              40


<210>    31
<211>    40
<212>    DNA
<213>    Artificial Sequence

<220>
<223>    Synthetic (Random aptamer)


<400>    31
ccccctcagg attttccgta gcttgtctgg cagcagctgt                              40


<210>    32
<211>    40
<212>    DNA
<213>    Artificial Sequence

<220>
<223>    Synthetic (Random aptamer)


<400>    32
tggcatttaa taaacaccct cgttaacgcg tgtgtggatt                              40


<210>    33
<211>    40
<212>    DNA

<213>    Artificial Sequence

<220>
<223>    Synthetic (Random aptamer)


<400>    33
gcagctccat tgaggctaga acgtgagtcg taccccacca                              40


<210>    34
<211>    40
<212>    DNA
<213>    Artificial Sequence

<220>
<223>    Synthetic (Random aptamer)


<400>    34
cgctattggg taaaggatct ctcgtagctc tgcatgagcc                              40


<210>    35
<211>    40
<212>    DNA
<213>    Artificial Sequence

<220>
<223>    Synthetic (Random aptamer)


<400>    35
gctggtgtgg ccgccggaag atgacttata ccctgcactc                              40


<210>    36
<211>    40
<212>    DNA
<213>    Artificial Sequence

<220>
<223>    Synthetic (Random aptamer)


<400>    36
cttttttcca aaccgggtgt gccacggcct gcgaatcggt                              40


<210>    37
<211>    40
<212>    DNA
<213>    Artificial Sequence

<220>
<223>    Synthetic (Random aptamer)


<400>    37
tgggaagaaa tcaatgaatg tctcactgat agctggtggt                              40


<210>    38

<211>    40
<212>    DNA
<213>    Artificial Sequence

<220>
<223>    Synthetic (Random aptamer)


<400>    38
tcccgatttg atggggtgct gcttatgata gtcacgaaac                              40


<210>    39
<211>    40
<212>    DNA
<213>    Artificial Sequence

<220>
<223>    Synthetic (Random aptamer)


<400>    39
aggcccgttt ttacctactc tcatatttgg acaaccgggt                              40


<210>    40
<211>    40
<212>    DNA
<213>    Artificial Sequence

<220>
<223>    Synthetic (Random aptamer)


<400>    40
ttatttgtcc atacattata gcgcgacgtg gaacaggtaa                              40


<210>    41
<211>    288
<212>    PRT
<213>    Artificial Sequence

<220>
<223>    Synthetic (TGFBRII ectodomain)


<400>    41
Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Leu Ala Gly
            100                 105                 110

Ser Gly Ser Gly His Met His His His His His His Ser Ser Gly Leu
        115                 120                 125

Val Pro Arg Gly Ser Gly Met Lys Gln Thr Ala Ala Ala Lys Phe Gly
    130                 135                 140

Arg Gln His Met Asp Ser Pro Asp Leu Asn Asp Met Ile Val Thr Asp
145                 150                 155                 160

Asn Asn Gly Ala Val Lys Phe Pro Gln Leu Cys Lys Phe Cys Asp Val
                165                 170                 175

Arg Phe Ser Thr Cys Asp Asn Gln Lys Ser Cys Met Ser Asn Cys Ser
            180                 185                 190

Ile Thr Ser Ile Cys Glu Lys Pro Gln Glu Val Cys Val Ala Val Trp
        195                 200                 205

Arg Lys Asn Asp Glu Asn Ile Thr Leu Glu Thr Val Cys His Asp Pro
    210                 215                 220

Lys Leu Pro Tyr His Asp Phe Ile Leu Glu Asp Ala Ala Ser Pro Lys
225                 230                 235                 240

Cys Ile Met Lys Glu Lys Lys Lys Pro Gly Glu Thr Phe Phe Met Cys
                245                 250                 255

Ser Cys Ser Ser Asp Glu Cys Asn Asp Asn Ile Ile Phe Ser Glu Glu
            260                 265                 270

Tyr Asn Thr Ser Asn Pro Asp Leu Leu Glu His His His His His His
        275                 280                 285

<210>   42
<211>   171
<212>   PRT
<213>   Artificial Sequence

<220>
<223>   Synthetic (VEGF ectodomain)

<400>   42
His His His His His His Ala Pro Met Ala Glu Gly Gly Gly Gln Asn
1               5                   10                  15

His His Glu Val Val Lys Phe Met Asp Val Tyr Gln Arg Ser Tyr Cys
            20                  25                  30

His Pro Ile Glu Thr Leu Val Asp Ile Phe Gln Glu Tyr Pro Asp Glu
        35                  40                  45

Ile Glu Tyr Ile Phe Lys Pro Ser Cys Val Pro Leu Met Arg Cys Gly
    50                  55                  60

Gly Cys Cys Asn Asp Glu Gly Leu Glu Cys Val Pro Thr Glu Glu Ser
65                  70                  75                  80

Asn Ile Thr Met Gln Ile Met Arg Ile Lys Pro His Gln Gly Gln His
                85                  90                  95

Ile Gly Glu Met Ser Phe Leu Gln His Asn Lys Cys Glu Cys Arg Pro
            100                 105                 110

Lys Lys Asp Arg Ala Arg Gln Glu Asn Pro Cys Gly Pro Cys Ser Glu
        115                 120                 125

Arg Arg Lys His Leu Phe Val Gln Asp Pro Gln Thr Cys Lys Cys Ser
    130                 135                 140

Cys Lys Asn Thr Asp Ser Arg Cys Lys Ala Arg Gln Leu Glu Leu Asn
145                 150                 155                 160

Glu Arg Thr Cys Arg Cys Asp Lys Pro Arg Arg
                165                 170


<210>    43
<211>    480
<212>    PRT
<213>    Artificial Sequence

<220>
<223>    Syntheric (CBF1)


<400>    43
Met Ala Trp Ile Lys Arg Lys Phe Gly Glu Arg Pro Pro Pro Lys Arg
1               5                   10                  15

Leu Thr Arg Glu Ala Met Arg Asn Tyr Leu Lys Glu Arg Gly Asp Gln
            20                  25                  30

Thr Val Leu Ile Leu His Ala Lys Val Ala Gln Lys Ser Tyr Gly Asn
        35                  40                  45

Glu Lys Arg Phe Phe Cys Pro Pro Pro Cys Val Tyr Leu Met Gly Ser
    50                  55                  60

Gly Trp Lys Lys Lys Lys Glu Gln Met Glu Arg Asp Gly Cys Ser Glu
65                  70                  75                  80

Gln Glu Ser Gln Pro Cys Ala Phe Ile Gly Ile Gly Asn Ser Asp Gln
                85                  90                  95

Glu Met Gln Gln Leu Asn Leu Glu Gly Lys Asn Tyr Cys Thr Ala Lys
            100                 105                 110

Thr Leu Tyr Ile Ser Asp Ser Asp Lys Arg Lys His Phe Met Leu Ser
        115                 120                 125

Val Lys Met Phe Tyr Gly Asn Ser Asp Asp Ile Gly Val Phe Leu Ser
    130                 135                 140

Lys Arg Ile Lys Val Ile Ser Lys Pro Ser Lys Lys Lys Gln Ser Leu
145                 150                 155                 160

Lys Asn Ala Asp Leu Cys Ile Ala Ser Gly Thr Lys Val Ala Leu Phe
                165                 170                 175

Asn Arg Leu Arg Ser Gln Thr Val Ser Thr Arg Tyr Leu His Val Glu
            180                 185                 190

Gly Gly Asn Phe His Ala Ser Ser Gln Gln Trp Gly Ala Phe Phe Ile
        195                 200                 205

His Leu Leu Asp Asp Asp Glu Ser Glu Gly Glu Glu Phe Thr Val Arg
    210                 215                 220

Asp Gly Tyr Ile His Tyr Gly Gln Thr Val Lys Leu Val Cys Ser Val
225                 230                 235                 240

Thr Gly Met Ala Leu Pro Arg Leu Ile Ile Arg Lys Val Asp Lys Gln
                245                 250                 255

Thr Ala Leu Leu Asp Ala Asp Asp Pro Val Ser Gln Leu His Lys Cys
            260                 265                 270

Ala Phe Tyr Leu Lys Asp Thr Glu Arg Met Tyr Leu Cys Leu Ser Gln
        275                 280                 285

Glu Arg Ile Ile Gln Phe Gln Ala Thr Pro Cys Pro Lys Glu Pro Asn
    290                 295                 300

Lys Glu Met Ile Asn Asp Gly Ala Ser Trp Thr Ile Ile Ser Thr Asp
305                 310                 315                 320

Lys Ala Glu Tyr Thr Phe Tyr Glu Gly Met Gly Pro Val Leu Ala Pro
                325                 330                 335

Val Thr Pro Val Pro Val Val Glu Ser Leu Gln Leu Asn Gly Gly Gly
            340                 345                 350

Asp Val Ala Met Leu Glu Leu Thr Gly Gln Asn Phe Thr Pro Asn Leu
        355                 360                 365

Arg Val Trp Phe Gly Asp Val Glu Ala Glu Thr Met Tyr Arg Cys Gly
    370                 375                 380

Glu Ser Met Leu Cys Val Val Pro Asp Ile Ser Ala Phe Arg Glu Gly
385                 390                 395                 400

Trp Arg Trp Val Arg Gln Pro Val Gln Val Pro Val Thr Leu Val Arg
                405                 410                 415

Asn Asp Gly Ile Ile Tyr Ser Thr Ser Leu Thr Phe Thr Tyr Thr Pro
            420                 425                 430

Glu Pro Gly Pro Arg Pro His Cys Ser Ala Ala Gly Ala Ile Leu Arg
        435                 440                 445

Ala Asn Ser Ser Gln Val Pro Pro Asn Glu Ser Asn Thr Asn Ser Glu
    450                 455                 460

Gly Ser Tyr Thr Asn Ala Ser Thr Leu Glu His His His His His His
465                 470                 475                 480


<210>    44
<211>    40

<212>    DNA
<213>    Artificial Sequence

<220>
<223>    Synthetic (Nucleic acid construct)


<220>
<221>    misc_feature
<222>    (1)..(3)
<223>    each n is A, T, G or C


<220>
<221>    misc_feature
<222>    (9)..(15)
<223>    each n is A, T, G or C


<220>
<221>    misc_feature
<222>    (20)..(40)
<223>    each n is A, T, G or C


<400>    44
nnnggacgnn nnnnngtccn nnnnnnnnnn nnnnnnnnnn                                40


<210>    45
<211>    40
<212>    RNA
<213>    Artificial Sequence

<220>
<223>    Synthetic (Nucleic acid construct)


<220>
<221>    misc_feature
<222>    (1)..(3)
<223>    each n is A, U, G or C


<220>
<221>    misc_feature
<222>    (9)..(15)
<223>    each n is A, U, G or C


<220>
<221>    misc_feature
<222>    (20)..(40)
<223>    each n is A, U, G or C


<400>    45
nnnggacgnn nnnnnguccn nnnnnnnnnn nnnnnnnnnn                                40


<210>    46
<211>    86
<212>    DNA

<213>    Artificial Sequence

<220>
<223>    Synthetic (HFL Aptamer)


<220>
<221>    misc_feature
<222>    (24)..(26)
<223>    each n is A, T, G or C


<220>
<221>    misc_feature
<222>    (32)..(38)
<223>    each n is A, T, G or C


<220>
<221>    misc_feature
<222>    (43)..(63)
<223>    each n is A, T, G or C


<400>    46
tagggaagag aaggacatat gatnnnggac gnnnnnnngt ccnnnnnnnn nnnnnnnnnn        60

nnnttgacta gtacatgacc acttga                                              86


<210>    47
<211>    86
<212>    DNA
<213>    Artificial Sequence

<220>
<223>    Synthetic (HFL Aptamer)


<220>
<221>    misc_feature
<222>    (24)..(63)
<223>    each n is A, T, G or C


<400>    47
tagggaagag aaggacatat gatnnnnnnn nnnnnnnnnn nnnnnnnnnn nnnnnnnnnn        60

nnnttgacta gtacatgacc acttga                                              86

## Description

### TECHNICAL FIELD

The present invention relates to a novel nucleic acid structure, its use as an aptamer template, and a method for preparing an aptamer using thereof.

### BACKGROUND ART

Aptamers mean short length nucleic acid (RNA or DNA) molecules which bind to a certain target molecule with high affinity and specificity. Typical aptamers are about 20-80 nucleotides length, and the molecular weight is about 6-30kDa. Aptamers form a particular tertiary structure, and by this structure, they can recognize a target molecule, and they bind to a target material by interaction similar to forming a complex by binding of antibody-antigen structurally. Due to such characteristics, aptamers have been paid attention as various molecular tools related to researches of therapy of diseases related to biosensors, drug screening, and the like or therapeutic materials themselves.

In addition, aptamers have competitive advantages superior to antibodies: At first, in aspect of immunogenicity, it has been demonstrated that aptamers show acceptable toxicity when administered into a body, while do not show immunogenicity. Since general immune responses do not recognize fragments of nucleic acids, it is very difficult to induce antibodies against aptamers. Second, in aspect of stability, aptamers have high resistance to relatively high temperatures (95°C or higher,) and show high stability in circulation of normalization and subtraction processes. Furthermore, aptamers are easy to transfer in a lyophilized state, and can be stored at room temperature for a long period. Third, aptamers have an advantage capable of binding potentially to various and broad targets such as ion materials, organic or inorganic molecules, peptides, proteins, toxins, virus particles, whole cells, entire organs, living animals, and the like, in addition to nucleic acid molecules. Fourth, aptamers have an ability to distinguish two nucleic acid molecules that differ only by one nucleotide or conformational isomers. In addition, aptamers have convenience and availability of chemical modification, and they can be utilized by modification of various type, for example, sugar, backbone, base and other modification, and the like.

Nucleic acid-based medicines such as aptamers may be degraded by various enzymes in vivo such as nuclease, and the like when injected into a body, and therefore the pharmacological effect may be reduced. Due to such a reason, various modification for enhancing in vivo stability of nucleic acids has been researched. However, to provide modification for nucleic acids, additional costs are required, and also forms and physical properties of nucleic acids are affected all, and therefore, side effects such as increase of toxicity, degradation of binding ability to target molecules, and the like may be caused. In addition, the stability of the tertiary structure of aptamers is low, and thus when prepared as medicines, they have a structural disadvantage that the structure is unfolded at 37°C which is the temperature in vivo and they may be under denaturation at the end.

As a general selection method of aptamers, 'SELEX (Systematic Evolution of Ligands by Exponential Enrichment)' has been widely used (See U.S. patent No. 5,475,096 and No. 5,270,163, the documents are incorporated herein by reference).

SELEX is a process of drawing aptamers targeting a specific molecule in an oligonucleotide library that is a large scale of random sequences. In SELEX, the oligonucleotide library is reacted with the target molecule, and sequences which do not bind to the target molecule are removed, and sequences which bind to the target molecule are separated and amplified through PCR (polymerase chain reaction), and then the aforementioned process is repeated many times, and finally, only the sequences which bind to the target molecule strongest are selected.

The typical SELEX process has several limitations. Any aptamer in the random sequences in the library may not bind to a target molecule strongly. In addition, a lot of time is taken in the SELEX process, and it requires shortly from a few days up to several months. Additional adjustment may be required to reduce the length of the sequence of the aptamer or provide biological properties, since the finally separated aptamer has a low functional ability. In addition, in case of a random sequence library generally used in the SELEX process, there is a high possibility that sequences which do not form a tertiary structure are included, and thus the screening efficiency may decrease.

Accordingly, there is a need to improve the process for avoiding such disadvantages of SELEX and selecting a more useful and effective aptamer sequence.

### Detailed Description of the Invention

### TECHNICAL PROBLEM

Herein, provided are a novel structure of nucleic acid structure and a technology for preparing an aptamer with excellent stability and binding ability to a target molecule, by using for aptamer selection using the same as an aptamer template.

One embodiment provides a nucleic acid structure represented by the following general formula 1:

5'-[T1][S1][L][S2][T2]-3' (general formula 1)

wherein,
L site is a site forming a loop structure, and
S1 site and S2 site are sites forming a stem structure, and have anti-parallel complementary sequences (namely, the sequence from 5' to 3' direction of S1 and the sequence from 3' to 5' direction of S2 are complementary each other), and
T1 site represents a 5' end region and T2 represents a 3' end region.

The nucleotide may be each independently selected from A, T/U (meaning T or U, same as below), G, and C.

In one embodiment, the nucleic acid structure may be represented by the following general formula 2:

5'-[(N)ₚ][(X)₁][G(N)ₙ][(X)ₘ][(N)_{q}]-3' (general formula 2)

wherein,
N and X mean nucleotides selected from A, T/U, G, and C, and N is each independently selected from A, T/U, G, and C randomly, and X is each independently selected from A, T/U, G, and C randomly, and the ratio of C or G is 55% or higher (the upper limit is 100% or less than 100%) based on the total nucleotides of [(X)ₗ] or [(X)ₘ], and
[G(N)ₙ] is a site forming a loop structure, and n is the number of any nucleotide N, and may be an integer of 3 to 29, that is, 3, 4, 5, 6, 7, 8, 9, 10, 11, 12, 13, 14, 15, 16, 17, 18, 19, 20, 21, 22, 23, 24, 25, 26, 27, 28, or 29, and
[(X)ₗ] and [(X)ₘ] are sites forming a stem structure by forming a base pair with antiparallel complementary sequences each other, and 1 and m are numbers of any nucleotide X and each may be an integer of 3 to 10, for example, 3, 4, 5, 6, 7, 8, 9, or 10, and may be integers the same or different each other, for example, the same integers each other, and
[(N)ₚ] and [(N)_{q}] are 5' end region and 3' end region, respectively, and p and q are numbers of any nucleotide N, and each is independently an integer of 1 to 50 (integers the same or different each other), but not limited thereto.

In one embodiment, the nucleic acid structure may be represented by the following general formula 3, but not limited thereto:
NNN**GGAC**GNN NNNNN**GTCC**N NNNNNNNNNN (general formula 3; SEQ ID NO: 44)
NNN**GGAC**GNN NNNNN**GUCC**N NNNNNNNNNN (general formula 4; SEQ ID NO: 45)
(in the general formulas, N is each independently selected from A, T/U, G, and C, and the bolded part has anti-parallel complementary sequences and forms a stem structure, and the underlined part forms a loop structure)

The nucleic acid structure may further comprise a labeling material (fluorescent material, ligand (biotin, etc.)) and/or 1 to 30, 1 to 28, 1 to 25, 1 to 23, 1 to 20, 1 to 15, or 1 to 10 additional oligonucleotides (for example, may be a primer binding site) at the 5' end and/or 3' end, for amplification (for example, performing PCR), purification, and the like.

Other embodiment provides an aptamer template comprising the nucleic acid structure.

Other embodiment provides a composition for selecting or preparing a target material-specific binding nucleic acid molecule comprising the nucleic acid structure. The target material-specific binding nucleic acid molecule may be an aptamer.

Other embodiment provides a method for selecting or a method for preparing a target material-specific binding nucleic acid molecule using the nucleic acid structure.

The method for selecting or method for preparing may comprise
(1) contacting the nucleic acid structure with a target material; and
(2) measuring whether to bind between the nucleic acid structure and target material, and
may further comprise (3) determining the nucleic acid structure as a nucleic acid molecule specifically binding to the target material, when binding between the nucleic acid structure and target material is confirmed.

Other embodiment provides a method for selecting or a method for preparing a target material-specific binding nucleic acid aptamer using the nucleic acid structure.

The method for selecting or the method for preparing a nucleic acid aptamer may comprise
(a) contacting the nucleic acid structure with a target material; and
(b) measuring whether to bind between the nucleic acid structure and target material, and
may further comprise (c) determining the nucleic acid structure as a nucleic acid aptamer specifically binding to the target material, when binding between the nucleic acid structure and target material is confirmed.

In the methods, the nucleic acid structure used may be two or more kinds having different sequences each other at any nucleotide (N) position of general formulas 1 to 3.

The method for selecting or method for preparing a nucleic acid aptamer may be applied to the conventional SELEX method. In this case, the method for selecting or method for preparing a nucleic acid aptamer may comprise
(i) contacting a library comprising one or more kinds of the nucleic acid structures with beads in which a target material is immobilized;
(ii) washing the beads of the step (i); and
(iii) confirming the nucleic acid structure bound to the beads of the step (ii), and
may further comprise (iv) determining the nucleic acid structure confirmed in the step (iii) as a nucleic acid aptamer specifically binding to the target material.

Other embodiment provides a nucleic acid aptamer produced from the nucleic acid structure (aptamer template). Other embodiment provides a nucleic acid aptamer prepared by the method for preparing a nucleic acid aptamer. The nucleic acid aptamer may have the same nucleic sequence as the nucleic acid structure and bind specifically to a target material.

### TECHNICAL SOLUTION

Herein, it is suggested that the stability and binding ability to target molecules of aptamers can be enhanced by randomly providing a stem-loop structure at the starting region of the nucleic acid aptamer structure.

The technology provided herein relates to a technology of design or manufacturing of a nucleic acid aptamer, and more specifically, relates to a technology to allow selection of aptamers with excellent binding ability to target molecules and stability, by introducing a stem-loop structure to an aptamer library. In addition, the process of the technology provided herein is simple and can be easily performed, and therefore there is an advantage of low efforts and costs.

One embodiment provides a nucleic acid structure represented by the following general formula 1:

5'-[T1][S1][L][S2][T2]-3' (general formula 1)

wherein,
L site is a site forming a loop structure, and may comprise 4 to 30, 4 to 28, 4 to 25, 4 to 23, 4 to 20, 4 to 18, 4 to 15, 4 to 13, 4 to 10, 5 to 30, 5 to 28, 5 to 25, 5 to 23, 5 to 20, 5 to 18, 5 to 15, 5 to 13, 5 to 10, 6 to 30, 6 to 28, 6 to 25, 6 to 23, 6 to 20, 6 to 18, 6 to 15, 6 to 13, 6 to 10, or about 8 nucleotides, and
S1 site and S2 site are sites forming a stem structure, and have anti-parallel complementary sequences (namely, the sequence from 5' to 3' direction of S1 and the sequence from 3' to 5' direction of S2 are complementary each other) and may comprise 3 to 10, 3 to 8, 3 to 5, 4 to 10, 4 to 8, 4 to 5, or about 4 nucleotides, respectively, and S1 and S2 may comprise the same number of nucleotides, and
T1 site represents a 5' end region (namely, extended site to the 5' end of the S1 site forming a stem structure) and T2 site represents a 3' end region (namely, extended site to the 3' end of the S2 site forming a stem structure), and each independently, may comprise 1 to 50, 1 to 45, 1 to 40, 1 to 35, 1 to 30, 1 to 28, 1 to 25, 1 to 23, 1 to 20, 1 to 15, 1 to 10, 1 to 5, 2 to 50, 2 to 45, 2 to 40, 2 to 35, 2 to 30, 2 to 28, 2 to 25, 2 to 23, 2 to 20, 2 to 15, 2 to 10, 2 to 5, 3 to 50, 3 to 45, 3 to 40, 3 to 35, 3 to 30, 3 to 28, 3 to 25, 3 to 23, 3 to 20, 3 to 15, 3 to 10, 3 to 5, 5 to 50, 5 to 45, 5 to 40, 5 to 35, 5 to 30, 5 to 28, 5 to 25, 5 to 23, 5 to 20, 5 to 15, 5 to 10, 10 to 50, 10 to 45, 10 to 40, 10 to 35, 10 to 30, 10 to 28, 10 to 25, 10 to 23, 10 to 20, 10 to 15, 15 to 50, 15 to 45, 15 to 40, 15 to 35, 15 to 30, 15 to 28, 15 to 25, 15 to 23, 15 to 20, 17 to 50, 17 to 45, 17 to 40, 17 to 35, 17 to 30, 17 to 28, 17 to 25, 17 to 23, 17 to 20, 20 to 50, 20 to 45, 20 to 40, 20 to 35, 20 to 30, 20 to 28, 20 to 25, or 20 to 23 nucleotides, and T1 and T2 may comprise a different number of nucleotides, respectively.

The nucleotide may be each independently selected from A, T/U, G, and C.

In one embodiment, the L site may necessarily comprise guanine (G) at the 5' end, and for example, it may be represented as follows:
5'-G(N)ₙ-3' (N represents a nucleotide selected from A, G, C, and T/U (when 2 or more of nucleotides are comprised, they may be the same or different each other), n represents the number of nucleotides comprised in N, and it may be an integer of 3 to 29, that is, 3, 4, 5, 6, 7, 8, 9, 10, 11, 12, 13, 14, 15, 16, 17, 18, 19, 20, 21, 22, 23, 24, 25, 26, 27, 28, or 29, for example, 7, but not limited thereto). In one embodiment, the L site may have the sequence of 5'-GAAAAAAA-3', but not limited thereto.

In one embodiment, the S1 site and S2 site have anti-parallel complementary sequences (namely, the sequence from 5' to 3' direction of S1 and the sequence from 3' to 5' direction of S2 are complementary each other) to form a stem structure, and form base pairs. Then, the binding ability of GC base pair is stronger than the binding ability of AT/U base pair, and therefore, the ratio of G or C sequence in the total sequence or the ratio of GC base pair of the total base pairs may be 55% or higher, 57% or higher, 60% or higher, 62% or higher, 66% or higher, 70% or higher, 71% or higher, 75% or higher, 77% or higher, 80% or higher, 83% or higher, 85% or higher, 87% or higher, or 88% or higher (the upper limit is 100% or less than 100%). Although not limited thereto, in order to avoid excessive presence of CG base pairs in the stem structure, the case where all nucleotides of the S1 site and S2 site are C or G may be excluded herein. In other words, the S1 site and S2 site may comprise at least one or more of A or T/U (but, it is not over 25%, 33% or 40% of the total S1 site and S2 site sequences), and then, A or T/U may be positioned 1, 2 or 3 nucleotides away from the L site, but not limited thereto. In one embodiment, the S1 site may have the sequence of 5'-GGAC-3' and the S2 site may have the sequence of 5'-GT/UCC-3', but not limited thereto.

T1 site is the 5' end region located adj acent to 5'of S1 (extended site of 5' end of S1), and may comprise 1 to 50, 1 to 45, 1 to 40, 1 to 35, 1 to 30, 1 to 28, 1 to 25, 1 to 23, 1 to 20, 1 to 15, 1 to 10, 1 to 5, 2 to 50, 2 to 45, 2 to 40, 2 to 35, 2 to 30, 2 to 28, 2 to 25, 2 to 23, 2 to 20, 2 to 15, 2 to 10, 2 to 5, 3 to 50, 3 to 45, 3 to 40, 3 to 35, 3 to 30, 3 to 28, 3 to 25, 3 to 23, 3 to 20, 3 to 15, 3 to 10, or 3 to 5 nucleotides the same or different each other. T2 site is the 3' end region located adjacent to 3' of S2 (extended site of 3' end of S2), and may comprise 5 to 50, 5 to 45, 5 to 40, 5 to 35, 5 to 30, 5 to 28, 5 to 25, 5 to 23, 5 to 20, 5 to 15, 5 to 10, 10 to 50, 10 to 45, 10 to 40, 10 to 35, 10 to 30, 10 to 28, 10 to 25, 10 to 23, 10 to 20, 10 to 15, 15 to 50, 15 to 45, 15 to 40, 15 to 35, 15 to 30, 15 to 28, 15 to 25, 15 to 23, 15 to 20, 17 to 50, 17 to 45, 17 to 40, 17 to 35, 17 to 30, 17 to 28, 17 to 25, 17 to 23, 17 to 20, 20 to 50, 20 to 45, 20 to 40, 20 to 35, 20 to 30, 20 to 28, 20 to 25, or 20 to 23 nucleotides the same or different each other.

In one embodiment, the nucleic acid structure provided herein may have a structure in which a stem-loop structure is biased toward the 5' end. In other words, in the nucleic acid structure, the number of nucleotides of T1 that is the extended site of the 5' end of S1 in the stem structure may be less than the number of nucleotides of T2 that is the extended site of the 3' end of S2 in the stem structure (T1 number < T2 number). For example, while satisfying the condition that the number of nucleotides of T2 exceeds the number of nucleotides of T1, T1 may comprise 1 to 20, 1 to 15, 1 to 10, 1 to 5, 2 to 20, 2 to 15, 2 to 10, 2 to 5, 3 to 20, 3 to 15, 3 to 10, 3 to 5, 5 to 20, 5 to 15, 5 to 10, 10 to 20, 10 to 15, 15 to 20, or 4 nucleotides, and T2 may comprise 15 to 50, 15 to 45, 15 to 40, 15 to 35, 15 to 30, 15 to 28, 15 to 25, 15 to 23, 15 to 20, 17 to 50, 17 to 45, 17 to 40, 17 to 35, 17 to 30, 17 to 28, 17 to 25, 17 to 23, 17 to 20, 20 to 50, 20 to 45, 20 to 40, 20 to 35, 20 to 30, 20 to 28, 20 to 25, or 21 nucleotides.

In one embodiment, the nucleic acid structure may be represented by the following general formula 2:

5'-[(N)ₚ][(X)ₗ][G(N)ₙ][(X)ₘ][(N)_{q}]-3' (general formula 2)

wherein,
N and X mean a nucleotide selected from A, T/U, G, and C, and N is each independently selected from A, T/U, G, and C randomly, and X is each independently selected from A, T/U, G, and C randomly, and the ratio of C or G is 55% or higher, 57% or higher, 60% or higher, 62% or higher, 66% or higher, 70% or higher, 71% or higher, 75% or higher, 77% or higher, 80% or higher, 83% or higher, 85% or higher, 87% or higher, or 88% or higher (the upper limit is 100% or less than 100%) based on the total nucleotides of [(X)ₗ] or [(X)ₘ], and
[G(N)ₙ] is a site forming a loop structure, and n is the number of any nucleotide N, and may be an integer of 3 to 29, that is, 3, 4, 5, 6, 7, 8, 9, 10, 11, 12, 13, 14, 15, 16, 17, 18, 19, 20, 21, 22, 23, 24, 25, 26, 27, 28, or 29, and
[(X)ₗ] and [(X)ₘ] are sites forming a stem structure by having anti-parallel complementary sequences to form a base pair, and 1 and m are the numbers of any nucleotide X, and they may be an integer of 3 to 10, for example, 3, 4, 5, 6, 7, 8, 9, or 10, respectively, and they may be an integer the same or different each other, for example, an integer the same each other, and
[(N)ₚ] and [(N)_{q}] are 5' end region (extended site of the 5' end of S1) and 3' end region (extended site of the 3' end of S2), respectively, and p and q are the numbers of any nucleotide N, and may be each independently an integer of 1 to 50 (may be an integer the same or different each other), for example, 1, 2, 3, 4, 5, 6, 7, 8, 9, 10, 11, 12, 13, 14, 15, 16, 17, 18, 19, 20, 21, 22, 23,24,25,26,27,28,29,30,31,32,33,34,35,36,37,38,39,40,41,42,43,44,45,46,47,48, 49 or 50, respectively, and in one embodiment, p may be an integer of 1 to 30 (for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30), an integer of 1 to 20, an integer of 1 to 10, or an integer of 1 to 5, and q may be an integer of 10 to 50 (for example, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50), an integer of 15 to 50, or an integer of 20 to 50, but not limited thereto.

In one embodiment, p which is the number of nucleotides of the 5' end region of S1 (extended site of the 5' end of S1) may be smaller than q which is the number of nucleotides of the 3' end region of S2 (extended site of the 3' end of S2). In one embodiment, while satisfying the condition of p<q, p may be an integer of 1 to 30 (for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30), and q may be an integer of 10 to 50 (for example, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50), but not limited thereto.

In one embodiment, the nucleic acid structure may be represented by the following general formula 3 or general formula 4, but not limited thereto:
NNN**GGAC**GNN NNNNN**GTCC**N NNNNNNNNNN (general formula 3; SEQ ID NO: 44)
NNN**GGAC**GNN NNNNN**GUCC**N NNNNNNNNNN (general formula 4; SEQ ID NO: 45)
(in the general formulas, N is each independently selected from A, T/U (T in the general formula 3, U in the general formula 4), G, and C, and the bolded part has anti-parallel complementary sequences and forms a stem structure, and the underlined part forms a loop structure)

The nucleic acid structure may further comprise a labeling material (fluorescent material, ligand (biotin, etc.)) and/or 1 to 30, 1 to 28, 1 to 25, 1 to 23, 1 to 20, 1 to 15, 1 to 10, 2 to 30, 2 to 28, 2 to 25, 2 to 23, 2 to 20, 2 to 15, 2 to 10, 3 to 30, 3 to 28, 3 to 25, 3 to 23, 3 to 20, 3 to 15, 3 to 10, 5 to 30, 5 to 28, 5 to 25, 5 to 23, 5 to 20, 5 to 15, or 5 to 10 additional oligonucleotides (for example, may be a primer binding site) at the 5' end and/or 3' end, for amplification (for example, performing PCR), purification, and the like.

The nucleic acid structure provided herein may be a DNA structure and/or an RNA structure. The nucleic acid structure may be a non-naturally occurring material, and may be chemically or recombinantly synthesized.

The nucleotide disclosed herein is represented as comprised bases, and T/U means T for the DNA structure and U for the RNA structure.

The nucleic acid structure may have a unique stem-loop secondary structure. The nucleic acid structure of stem-loop secondary structure may form a double strand at the stem site and may be a single strand form at other parts. In addition, the nucleic acid structure may form a library having various sequences by inserting any nucleotide to N of the general formula 2 or 3. The nucleic acid structure of stem-loop structure in which any nucleotide is inserted to N of the general formula 2 or 3 may be bound to a desired target material (for example, nucleic acid molecule (DNA, RNA, etc.), protein, peptide, and the like), and thereby it may be usefully applied for selecting an structure having excellent binding ability.

As described above, the nucleic acids structure or aptamer obtained therefrom provided herein, may retain one or more of stem-loop structures, and induce an increase of Tm value and/or decrease of free energy value, to secure excellent structural stability compared to the case without comprising a stem-loop structure. In addition, the nucleic acid structure and aptamer obtained therefrom can hinder degradation by nuclease such as exonuclease, and the like, by having a stem-loop structure biased toward the 5' end, and therefore may secure excellent efficacy persistency in vivo (for example, in blood or in serum) and/or intracellular, compared to the nucleic acid structure not having such a structure (for example, the stem-loop structure is biased toward the center or 3' end).

The target material used herein is a biomaterial to be targeted, and there is no special limitation, and it may be one or more kinds selected from the group consisting of nucleic acid molecules such as DNA, RNA, etc., peptides, proteins, small molecule compounds, and the like, and may be isolated from a living body or cell.

In one specific embodiment, the target material
may be a biomaterial with a pI (isoelectric point) value (for example, may be measured by IPC (Isoelectric Point Calculator)) of about 2 to about 14, about 2 to about 13, about 2 to about 12, about 2 to about 11, about 2 to about 10, about 2 to about 9.5, about 2 to about 9, about 2 to about 8.75, about 2 to about 8.5, about 3 to about 14, about 3 to about 13, about 3 to about 12, about 3 to about 11, about 3 to about 10, about 3 to about 9.5, about 3 to about 9, about 3 to about 8.75, about 3 to about 8.5, about 4 to about 14, about 4 to about 13, about 4 to about 12, about 4 to about 11, about 4 to about 10, about 4 to about 9.5, about 4 to about 9, about 4 to about 8.75, about 4 to about 8.5, about 4.5 to about 14, about 4.5 to about 13, about 4.5 to about 12, about 4.5 to about 11, about 4.5 to about 10, about 4.5 to about 9.5, about 4.5 to about 9, about 4.5 to about 8.75, about 4.5 to about 8.5, about 5 to about 14, about 5 to about 13, about 5 to about 12, about 5 to about 11, about 5 to about 10, about 5 to about 9.5, about 5 to about 9, about 5 to about 8.75, about 5 to about 8.5, about 5.5 to about 14, about 5.5 to about 13, about 5.5 to about 12, about 5.5 to about 11, about 5.5 to about 10, about 5.5 to about 9.5, about 5.5 to about 9, about 5.5 to about 8.75, or about 5.5 to about 8.5, for example, a polypeptide comprising 2 or more of amino acids. When the target material is a polypeptide (for example, human protein), the polypeptide having a pI value in the above range may be confirmed through a well-known route (for example, see http://isoelectricpointdb.org/40/UP000005640_9606_all_isoelectnc_point_proteome_Homo_sap iens_Human.html). In general, it is common that the lower the pI value of the target material is, the weaker binding with aptamers is due to electrostatic repulsive power, but the aptamer selected using the nucleic acid structure provided herein has an advantage of high binding ability even for materials with a relatively low pI value (for example, proteins with a pI value of 7 or less, 6.8 or less, 6.5 or less, 6.2 or less, 6 or less, or 5.8 or less).

The target material may be a biomaterial having a weight average molecular weight of about 1 to about 1000 KDa, about 1 to about 500 KDa, about 1 to about 300 KDa, about 1 to about 100 KDa, about 1 to about 100 KDa, about 1 to about 90 KDa, about 1 to about 80 KDa, about 1 to about 70 KDa, about 1 to about 60 KDa, about 10 to about 1000 KDa, about 10 to about 500 KDa, about 10 to about 300 KDa, about 10 to about 100 KDa, about 10 to about 90 KDa, about 10 to about 80 KDa, about 10 to about 70 KDa, about 10 to about 60 KDa, about 15 to about 1000 KDa, about 15 to about 500 KDa, about 15 to about 300 KDa, about 15 to about 100 KDa, about 15 to about 90 KDa, about 15 to about 80 KDa, about 15 to about 70 KDa, or about 15 to about 60 KDa, for example, polypeptide but not limited thereto. For example, the target material may be one or more kinds selected from all polypeptides (two or more amino acids linked by peptide bonds; for example, proteins and/or peptides) which can act as a marker of physiological and/or pathological phenomena or show activity in a living body. For example, the target material may be one or more kinds selected from the group consisting of enzymes, hormones, growth factors, receptors, transport polypeptides, immune polypeptides (collectively refer to polypeptides made in immunocytes), signaling polypeptides, polypeptides composing living body, toxoproteins, immunogens of bacteria or viruses (surface protein antigens, etc.), and the like.

In one specific embodiment, the target material may be one or more kinds selected from the group consisting of
enzymes including hydrolase (for example, protease, phosphatase, etc.), oxidoreductase, transferase of uridine, methyl group, phosphate group (for example, uridylyl transferase (for example, Terminal Uridylyltransferase 7, etc.), kinase, etc.), and the like,
hormones including insulin, growth hormone, growth hormone releasing hormone, melatonin, serotonin, thyroid hormone, thyroid stimulating hormone, thyroid stimulating hormone releasing hormone, epinephrine, norepinephrine, dopamine, adiponectin, adrenocorticotropic hormone, adrenocorticotropic hormone releasing hormone, vasopressin, calcitonin, cholecystokinin, follicle stimulating hormone, gastrin, ghrelin, glucagon, human chorionic gonadotropin, luteinizing hormone, parathormone, prolactin, secretin, lipotropin, histamine, and the like,
growth factors including insulin-Like growth factors (IGFs), epidermal growth factor (EGF), VEGF (Vascular endothelial growth factor), Angiopoietin, nerve growth factor (NGF), Erythropoietin (EPO), Fibroblast growth factor (FGF), Platelet-derived growth factor (PDGF), Transforming growth factor (TGF), and the like,
receptors including G protein-coupled receptor (GPCR), receptor tyrosine kinase (RTK), ionotropic receptor, and the like,
transport polypeptides such as hemoglobin, transferrin, and the like,
immune polypeptides including immunoglobulin (for example, IgG (IgG1, IgG2, IgG3, IgG4), IgA, IgD, IgM IgE, etc.), cytokine (for example, interleukin such as IL-1, IL-2, IL-3, IL-4, IL-5, IL-6, IL-7, IL-8, IL-9, IL-10, IL-11, IL-12, IL-13, IL-14, IL-15, IL-16, IL-17, IL-18), tumor necrosis factor (TNF) such as TNF-alpha, beta or gamma, interferon(IFN)-alpha, -beta, - gamma, -omega or -tau, TRAIL (TNF-related apoptosis-inducing ligand), colony stimulating factor (CSF) (for example, G-CSF(Granulocyte-colony stimulating factor), GM-CSF(Granulocyte-macrophage colony-stimulating factor), M-CSF(macrophage colony-stimulating factor), etc.), and the like,
various kinds of signaling polypeptides such as extracellular matrix glycoprotein (for example, Reelin, etc.), Bone morphogenetic protein (BMP), and the like,
except for that, polypeptides composing living body such as collagen, elastin, keratin, tubulin, actin, fibrin, myosin, albumin, histone, casein, ovalbumin, and the like,
toxoproteins such as diphtheria, and the like,
surface protein antigens of viruses (for example, corona virus (e.g., MERS (Middle East respiratory syndrome) corona virus, etc.), influenza virus (for example, influenza type A (H1N1, H1N2, H2N2, H2N3, H3N1, H3N2, H3N8, H5N1, H5N2, H5N3, H5N6, H5N8, H5N9, H7N1, H7N2, H7N3, H7N4, H7N7, H7N9, H9N2, H10N7, etc.), influenza type B, influenza type C, etc.), measles virus, mumps virus, rubella virus, poxvirus, yellow fever virus, encephalitis virus, rabies virus, hepatitis A virus, hepatitis B virus, epidemic hemorrhagic fever virus, etc.) or bacteria (for example, tubercular bacillus, haemophilus pertussis, typhoid bacillus, cholera bacillus, pneumococcus, Clostridium tetani, etc.), and
transcription factors (TF) (for example, TATA box binding protein (TBP) such as TFIIA, TFIIB, TFIID, TFIIE, TFIIF, TFIIH, TFIIJ, etc., TBP associated factor (TAF), etc.), and the like.

Other embodiment provides an aptamer template comprising the nucleic acid structure.

Other embodiment provides a composition for selecting or preparing a target material-specific binding nucleic acid molecule comprising the nucleic acid structure. The target material-specific binding nucleic acid molecule may be an aptamer.

Other embodiment provides a method for selecting or a method for preparing a target material-specific binding nucleic acid molecule using the nucleic acid structure.

The method for selecting or method for preparing may comprise
(1) contacting the nucleic acid structure with a target material; and
(2) measuring whether to bind between the nucleic acid structure and target material, and
   may further comprise
(3) determining the nucleic acid structure as a nucleic acid molecule specifically binding to the target material, when binding between the nucleic acid structure and target material is confirmed.

Other embodiment provides a method for selecting or a method for preparing a nucleic acid aptamer specifically binding to a target material using the nucleic acid structure.

The method for selecting or method for preparing a nucleic acid aptamer may comprise
(a) contacting the nucleic acid structure with a target material; and
(b) measuring whether to bind between the nucleic acid structure and target material, and
   may further comprise
(c) determining the nucleic acid structure as a nucleic acid aptamer specifically binding to the target material, when binding between the nucleic acid structure and target material is confirmed.

In the methods, the used nucleic acid structure may be 2 or more kinds having a sequence different each other at the position of any nucleotide (N) of general formulas 1 to 3.

The method for selecting or method for preparing a nucleic acid aptamer may be applied for conventional SELEX method. In this case, the method for selecting or method for preparing a nucleic acid aptamer may comprise
(i) contacting a library comprising one or more kinds of nucleic acid structures with beads in which a target material is immobilized;
(ii) washing the beads of the step (i); and
(iii) confirming the nucleic acid structure bound to the beads of the step (ii), and
   may further comprise
(iv) determining the nucleic acid structure confirmed in the step (iii) as a nucleic acid aptamer specifically binding to the target material.

The method provided herein may be performed in vitro or in vivo, and for example, may be performed in vitro.

Other embodiment provides a nucleic acid aptamer produced from the nucleic acid structure (aptamer template). Other embodiment provides a nucleic acid aptamer prepared by the method for preparing a nucleic acid aptamer. As used herein, the aptamer template may mean a production frame in which certain nucleotide is inserted to any nucleotide (N position) to select and/or prepare a desired aptamer. Accordingly, the nucleic acid aptamer may have the same nucleic sequence as the nucleic acid structure (aptamer template) and be capable of binding to a target material specifically.

In one embodiment, the nucleic acid structure has an advantage of excellent binding ability to a target material and/or stability of a prepared aptamer, without comprising a modified nucleotide as any nucleotide, which is modified for enhancing binding ability to a target material and/or enhancing stability of an aptamer. In other embodiment, the nucleic acid structure may comprise a modified nucleotide to prepare an aptamer with further enhanced stability and/or binding ability. The modified nucleotide may be one or more kinds selected from the group consisting of 2'-position sugar modification, 5'-position pyrimidine modification (for example, 5-(N-benzylcarboxyamide)-2'-deoxyuridine, 5-(N-isobutylcarboxyamide)-2'-deoxyuridine, 5-(N-tryptaminocarboxylamide)-2'-deoxyuridine, 5-(N-[1-(3-trimethylammonium)propyl]carboxyamide)-2'-deoxyuridine chloride, 5-(N-naphthylmethylcarboxyamide)-2'-deoxyuridine, or 5-(N-[1-(2,3-dihydroxypropyl]carboxyamide)-2'-deoxyuridine), modification in exocyclic amines, substitution of 4-thiouridine, substitution of 5-bromo- or 5-iodo-uracil, backbone modification, methylation, and unusual base-pairing combinations such as isobasesisocytidine and isoguanidine, etc., but not limited thereto.

In one specific embodiment, by composing a stem part (S1 and S2 sites) of a stem-loop structure with 4 base pairs, a desired secondary structure (stem-loop structure) may be maintained even over the body temperature range (about 37 °C) (See Example 1-3). Then, for the base pairs forming the stem part, GC binding is more stable than AT/U binding, but AT base pair was inserted in the third stem part in order to avoid excessively high GC ratio. The loop part except for stem is a random sequence, but the sequence in which the loop starts is set to G, because designating G at this position further stabilizes the stem-loop structure.

### ADVANTAGEOUS EFFECTS

The technology provided herein relates to a Nucleic Acid Aptamer design technology, and it can be usefully applied for preparing an aptamer with enhanced binding ability to a target molecule and strengthened stability, using an aptamer library comprising nucleic acid structures in which a stem-loop structure is introduced. In addition, this technology is simple and technically easy, and also has an advantage of being able to prepare an aptamer at a low cost.

### BRIEF DESCRIPTION OF THE DRAWINGS

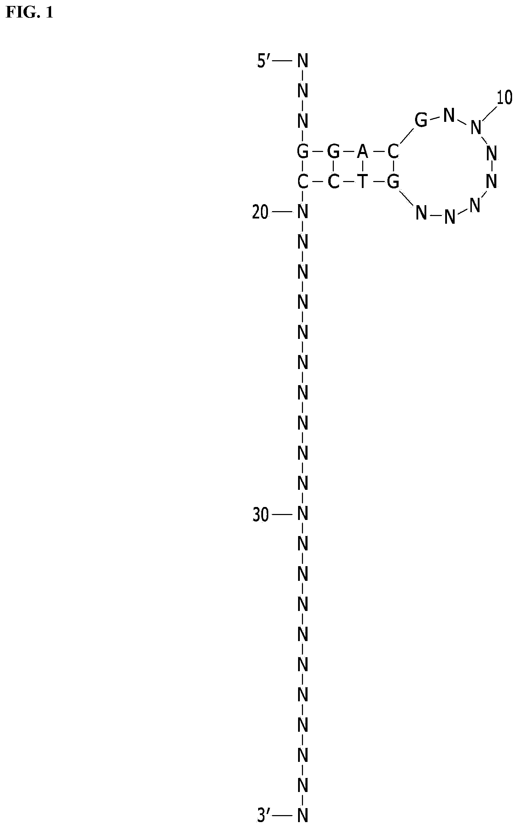

FIG. 1 schematically shows the structure of the aptamer template according to one example.
FIG. 2 is a graph showing the Tm value of the aptamer obtained by using the aptamer template (HFL) according to one example, by comparing with the Tm value of the aptamer obtained by using a random library.
FIG. 3 is a graph showing the free energy (ΔG) of the aptamer obtained by using the aptamer template (HFL) according to one example, by comparing with the free energy (ΔG) of the aptamer obtained by using a random library.
FIG. 4 is a graph showing binding of the VEGF-specific aptamer obtained by using the aptamer template (HFL) according to one example to VEGF (measured by the degree of luminescence) as a fold of the background value.
FIG. 5 is a graph showing binding of the CBF1-specific aptamer obtained by using the aptamer template (HFL) according to one example to CBF1 (measured by the degree of luminescence) as a fold of the background value.
FIG. 6 is a graph showing binding of the TGFBRII ectodomain-specific aptamer obtained by using the aptamer template (HFL) according to one example to TGFBRII ectodomain (measured by the degree of luminescence) as a fold of the background value.

### MODE FOR CARRYING OUT THE INVENTION

Hereinafter, the present invention will be described in more detail by examples, but they are illustrative and are not intended to limit the scope of the present invention. It is apparent to those skilled in the art that the examples described below can be modified without departing from the essential gist of the invention.

### Example 1: Aptamer library production

Based on a aptamer template of stem-loop structure having the following general formula, any base was introduced to N position to prepare a DNA aptamer library with a total length of 86mer (hereinafter, 'HFL (High Functional Library)'):
5'*-TAG GGA AGA GAA GGA CAT ATG AT* [NNN**GGAC**GNN NNNNN**GTCC**N NNNNNNNNNNNNNNNNNNNN] *TTG ACTAGT ACA TGA CCA CTT GA*-3' (general formula 5; SEQ ID NO: 46)
(in the general formula, N is each independently selected from A, T, G, and C, and the bolded part has anti-parallel complementary sequences and forms a stem structure, and the underlined part forms a loop structure; the part indicated in italics is an added sequence that can bind to forward and reverse primers).

For comparison, an oligonucleotide having the following general formula (86mer in total; hereinafter, 'Random library') was prepared:
5'-TAG GGA AGA GAA GGA CAT ATG AT [N]₄₀ TTG ACT AGT ACA TGA CCA CTT GA-3' (SEQ ID NO: 47; N is each independently selected from A, T, G, and C, and is different from the sequence in [] of the general formula 5)

All oligomers were produced in a form of Biotinylated-ssDNA(86mer) by commissioning to Bioneer corporation.

### Example 2: DNA aptamer analysis

For the ssDNA (aptamer) library prepared using HFL and Random library obtained in Example 1, PCR was performed using the following primers.
- Forward primer: 5'-biotin-TAG GGA AGA GAA GGA CAT ATG AT 3' (23mer)
- Reverse primer: 5'-TCA AGT GGT CAT GTA CTA GTC AA-3' (23mer)

The PCR process is summarized as follows:
(1) PCR mixture: Reverse primer(10uM), forward primer(10uM), dNTP(each 2mM), pfu(+) DNA buffer (CANCERROP Co., Ltd.), pfu(+) DNA polymerase(1 U; CANCERROP Co., Ltd.), and Library prepared in Example 1 (100ng).
(2) First cycles: 95°C for 3min, 53°C for 30sec, 72°C for 30min, 4°C hold.
(3) add the forward primer and mix.
(4) Second cycles: 95°C for 2min, start cycle (4 cycles)- 95°C for 30sec, 53°C for 30sec, 72°C for 10sec-end cycle, 72°C for 5min, 4°C hold.

For the obtained PCR products, cloning was performed using ToPcloner PCR Cloning kit (enzynomics Co., Ltd.). For cloning, pTOP Blunt V2 (10ng)1ul, TOPcloner buffer (1.2M NaCl, 0.06M MgCl₂)1ul, and PCR product(4ul) were mixed, and then it was left for 5 minutes at room temperature to prepare cloning product 6ul.

Using the prepared cloning product, DH5a chemically competent *E*. *coli* was transformed. More specifically, cloning product 6ul and DH5a chemically competent *E. coli* (CANCERROP Co., Ltd.) 5x10⁷ were mixed, and it was left in ice for 30 minutes and left at 42°C for 2 minutes, and then it was immediately put in ice. After that, the cells were put in SOC media (2.0% tryptone, 0.5% yeast extract, 10mM NaCl, 2.5mM KCl, 10mM MgCl2, 10mM MgSO4, 20mM Glucose) and mixed, and then they were left at 37°C for 1 hour, and then were under spin down at 5000xg for 2 minutes, and the supernatant was removed, and then SOC media 200ul was added again and mixed. The 5x10⁷ prepared cells were spread on 90π LB plate (+Ampicillin 50ug/ml) and then were cultured at 37°C overnight. The colony was picked and it was cultured at 37°C in a shaking incubator overnight.

To extract plasmid DNA in the cultured cells, Mini-prep was performed using mini-prep kit (CANCERROP Co., Ltd.). More specifically, the cell culture was centrifuged at 13000xg for 2 minutes to collect cells. The supernatant was removed and S1 buffer 250ul of mini-prep kit (CANCERROP Co., Ltd.) was added and mixed well, and then S2 buffer 250ul was added, and 4-6 times inverts were performed and S3 buffer 350ul was added and 6 times inverts were performed, and then it was centrifuged at 13000xg for 10 minutes and only the supernatant was collected and was loaded in a column, and then it was centrifuged at 13000rpm for 1 minute. PW buffer (CANCERROP Co., Ltd.) 700ul was loaded in a column, and then it was centrifuged at 13000rpm for 1 minutes twice, and then the column was put in a new tube, and D.W 50ul was loaded in the center of the column membrane, and it was left for 2 minutes, and then it was centrifuged at 13000rpm for 1 minute.

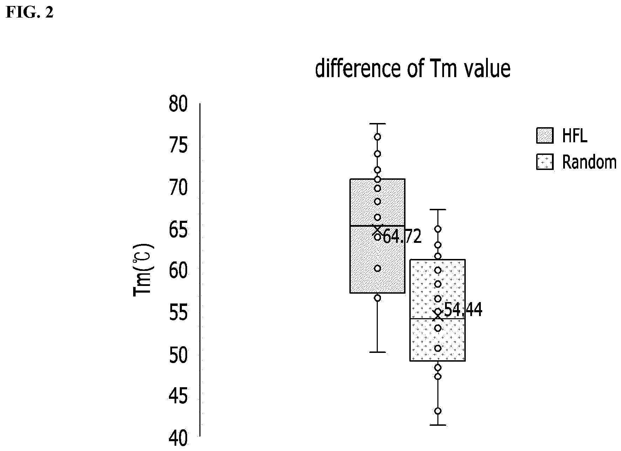

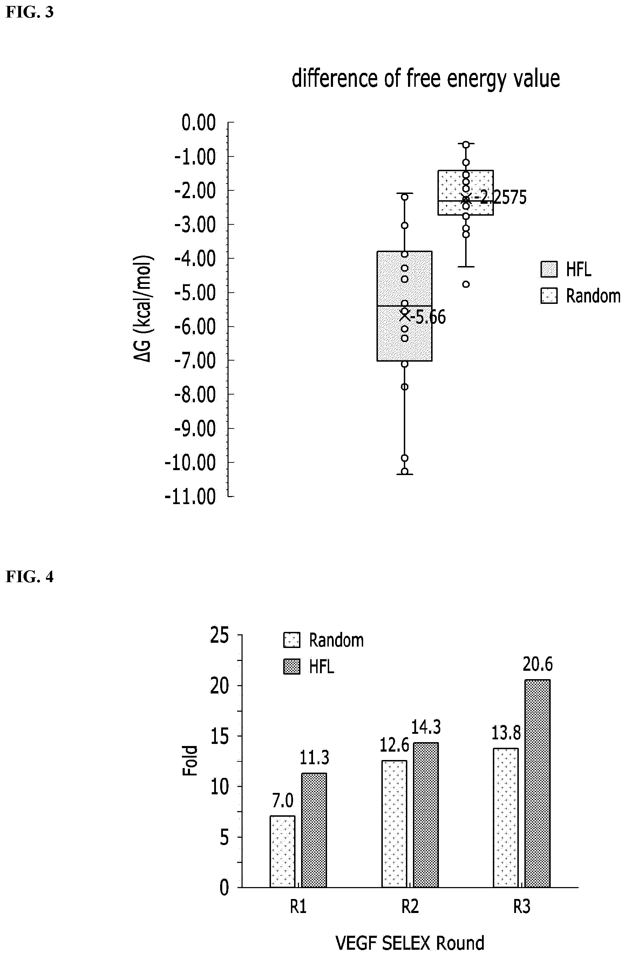

The sequence of DNA (20 aptamer DNAs selected in HFL and 20 aptamer DNAs obtained using Random library) obtained by the above method was analyzed (commissioned to Macrogen Co., Ltd.; reading the sequence with Universal M13 forward primer). In addition, free energy value and the Tm value of the aptamer section were obtained through 'Mfold' program (http://unafold.rna.albany.edu/?q=mfold) and the result of comparing the average value was shown in the following Tables 1 and 2, and FIG. 2 and FIG. 3.

**Table 1**

| Free energy (ΔG), Tm, and sequence of aptamers obtained using HFL. | | | |
|---|---|---|---|
| sequence | SEQ ID NO. | Tm (°C) | ΔG (kcal/mol) |
| ATAGGACGGATGAGCGTCCGGCCGGATGG GTTTTCCATCA | 1 | 77.3 | -10.29 |
| CTGGGACGCGGATGAGTCCGCTCCACCAG CCATCCATGTA | 2 | 66.2 | -6.35 |
| TTAGGACGCGAAGATGTCCTTGCAAGGGC TCATCGCATCA | 3 | 60.1 | -5.34 |
| ATGGGACGACCCTGTGTCCCTATATGCGCG GAGAGCCATC | 4 | 57.1 | -5.48 |
| CTTGGACGCCCTCGTGTCCCTTGCTCCTTC ACATACGCAA | 5 | 57.1 | -4.30 |
| GCTGGACGTTCGTGGGTCCCATTCTGAAGC CTAAGACTGG | 6 | 56.8 | -2.10 |
| GAGGGACGAACAAGCGTCCAGCGGTTATG CACCTTCGGTA | 7 | 64.2 | -6.07 |
| TACGGACGCACCTCCGTCCGGTTCTCAGCG TGGGGCGCAC | 8 | 75.8 | -10.33 |
| GCAGGACGTACTGATGTCCCGTTGCATGTC GCTAAGCTCC | 9 | 57.0 | -3.74 |
| CGCGGACGGGGTTTTGTCCCCCGTAGCTGA CTAAATACAT | 10 | 70.8 | -6.93 |
| ACGGGACGCGTTCTGGTCCCTACTTCTTGT GTGTCTATTC | 11 | 66.5 | -3.88 |
| AATGGACGCATCCCAGTCCCCCAAGCTTCC GTATCGTAAC | 12 | 56.5 | -2.20 |
| TGCGGACGCACGCCTGTCCTCAACCCATG ATTACACGTTT | 13 | 50.1 | -3.05 |
| TGGGGACGGGTATACGTCCATCTCGTATCC AACTAGGCGA | 14 | 63.9 | -5.59 |
| GTGGGACGCGTGTAGGTCCAAACGGTGCG GTTTGGCGCTC | 15 | 60.7 | -5.20 |
| GGAGGACGAGTGGTCGTCCCGCTGGGCAA TAGCTCGGGGC | 16 | 70.8 | -7.77 |
| ACAGGACGCCAGAGCGTCCAAGGTCGCCG CTAGTAGACCC | 17 | 73.8 | -9.86 |
| TCCGGACGTAGCCCGGTCCCAGCGCACGG TACCACCGTCG | 18 | 71.9 | -7.08 |
| CCAGGACGACTCTAGGTCCTCATGAGATT AGATATCGGTT | 19 | 68.1 | -3.02 |
| GTCGGACGGAATAACGTCCTAAATATTTAT GCAACGGGGA | 20 | 69.7 | -4.61 |
| average | | 64.72 | -5.66 |

**Table 2**

| Free energy(ΔG), Tm, and sequence of aptamers obtained using Random library. | | | |
|---|---|---|---|
| sequence | SEQ ID NO. | Tm (°C) | ΔG (kcal/mol) |
| CCTCTTGCGCGCTCCGTATCACATTTATATG ATGGCCAGT | 21 | 47.1 | -1.55 |
| TCTCTTACAACGGGTCTTGGCCATAACACA ATAATCCTGC | 22 | 50.9 | -1.11 |
| CCTAACTCGACCAGTGGGCTCGGACGGATG ATTAACCCTA | 23 | 48.2 | -1.19 |
| CCCCTACATCCCTTTTTGCCGCAAATCACC CAGCTCCTGA | 24 | 41.3 | -0.67 |
| CTTCCTTGATCGCTGCCCGATTTTTGGAATA GGTTCCTAG | 25 | 61.6 | -4.78 |
| CTCAGGTTGTCGATCTAACCATTGGCGGGT ACGTTGTACC | 26 | 56.4 | -4.24 |
| CCCATGTCAGCCGGTTAATCTTAATGTTGA TTCGAGTCAC | 27 | 43.0 | -0.63 |
| CTCTTGTCGCAGTACCTCAATTGAGCGTTG TACACATTGT | 28 | 55.1 | -1.74 |
| TGTATTTCAGTAATCCAACGGCACTTGTGA CACTGCTACT | 29 | 58.2 | -1.39 |
| TACTCAGACGAATCAAATATCGTGTCCAAA GTCCTCCGAG | 30 | 62.9 | -2.38 |
| CCCCCTCAGGATTTTCCGTAGCTTGTCTGG CAGCAGCTGT | 31 | 52.9 | -2.79 |
| TGGCATTTAATAAACACCCTCGTTAACGCG TGTGTGGATT | 32 | 50.5 | -1.96 |
| GCAGCTCCATTGAGGCTAGAACGTGAGTCG TACCCCACCA | 33 | 59.8 | -3.12 |
| CGCTATTGGGTAAAGGATCTCTCGTAGCTC TGCATGAGCC | 34 | 67.1 | -3.31 |
| GCTGGTGTGGCCGCCGGAAGATGACTTATA CCCTGCACTC | 35 | 61.6 | -2.43 |
| CTTTTTTCCAAACCGGGTGTGCCACGGCCT GCGAATCGGT | 36 | 53.3 | -2.28 |
| TGGGAAGAAATCAATGAATGTCTCACTGAT AGCTGGTGGT | 37 | 64.9 | -2.67 |
| TCCCGATTTGATGGGGTGCTGCTTATGATA GTCACGAAAC | 38 | 54.7 | -1.76 |
| AGGCCCGTTTTACCTACTCTCATATTTGGA CAACCGGGT | 39 | 48.4 | -2.46 |
| TTATTTGTCCATACATTATAGCGCGACGTG GAACAGGTAA | 40 | 50.9 | -2.69 |
| average | | 54.44 | -2.26 |

As shown in Tables 1 and 2, and FIG. 2 and FIG. 3, the aptamer obtained from the aptamer template having a stem-loop structure of the present invention (HFL), was confirmed to have higher Tm value and lower free energy value, compared to the aptamer obtained from the conventional random library, and this result shows that an aptamer with enhanced stability can be prepared without an additional stabilization process, using the aptamer template provided herein.

### Example 3: Protein SELEX using library - TGFBRII ectodomain, VEGF, & CBF1

pI value means the pH at which the net charge of protein becomes '0', and the value is determined by kinds of amino acids composing protein. Since an acidic amino acid has one more carboxyl group (-COOH) and a basic amino acid has one more amino group (-NH2), the more the acidic amino acid is in protein, the smaller the pI value is, and the more the basic amino acid is, the bigger the pI value is. When specific protein is present in a higher pH environment (for example, buffer) than its pI value, the protein has a negative charge (-), and in the lower pH environment (for example, buffer), it has a positive charge (+).

In the following example, three proteins belonging to different pI ranges (acidic/basic/hydrophobic), respectively, in the buffer environment of physiological pH 7.5 of human were selected to progress an experiment, and thereby it was confirmed that the HFL library of the present invention had high binding affinity to target protein than Random library in all different charge environments.

More specifically, using the DNA aptamer library prepared in Example 1 (Biotinylated-ssDNA (86mer) library), SELEX (Systematic evolution of ligands by exponential enrichment) was performed for the following three proteins:
TGFBRII ectodomain: N terminal 6x His-tagged, C terminal Thioredoxin-tagged, 35KDa, pi value: 5.74 
(MSDKIIHLTDDSFDTDVLKADGAILVDFWAEWCGPCKMIAPILDEIADEYQGKLTVAKL
NIDQNPGTAPKYGIRGIPTLLLFKNGEVAATKVGALSKGQLKEFLDANLALAGSGSGHM
HHHHHHSSGLVPRGSGMKQTAAAKFGRQHMDSPDLNDMIVTDNNGAVKFPQLCKFCD
VRFSTCDNQKSCMSNCSITSICEKPQEVCVAVWRKNDENITLETVCHDPKLPYHDFILED
AASPKCIMKEKKKPGETFFMCSCSSDECNDNIIFSEEYNTSNPDLLEHHHHHH; SEQ ID
NO: 41);
VEGF: Abcam Co., Ltd., N terminal 6X His-tagged VEGFA protein, 22KDa, pi value: 7.6 (HHHHHHAPMA EGGGQNHHEV VKFMDVYQRS YCHPIETLVD IFQEYPDEIE YIFKPSCVPL MRCGGCCNDE GLECVPTEES NITMQIMRIK PHQGQHIGEM SFLQHNKCEC RPKKDRARQE NPCGPCSERR KHLFVQDPQT CKCSCKNTDS RCKARQLELN ERTCRCDKPR R; SEQ ID NO: 42);
CBF1: C terminal 6X His-tagged, 54KDa, pi value: 8.3 (MAWIKRKFGE RPPPKRLTRE AMRNYLKERG DQTVLILHAK VAQKSYGNEK RFFCPPPCVY LMGSGWKKKK EQMERDGCSE QESQPCAFIG IGNSDQEMQQ LNLEGKNYCT AKTLYISDSD KRKHFMLSVK MFYGNSDDIG VFLSKRIKVI SKPSKKKQSL KNADLCIASG TKVALFNRLR SQTVSTRYLH VEGGNFHASS QQWGAFFIHL LDDDESEGEE FTVRDGYIHY GQTVKLVCSV TGMALPRLII RKVDKQTALL DADDPVSQLH KCAFYLKDTE RMYLCLSQER IIQFQATPCP KEPNKEMIND GASWTIISTD KAEYTFYEGM GPVLAPVTPV PVVESLQLNG GGDVAMLELT GQNFTPNLRV WFGDVEAETM YRCGESMLCV VPDISAFREG WRWVRQPVQV PVTLVRNDGI IYSTSLTFTY TPEPGPRPHC SAAGAILRAN SSQVPPNESN TNSEGSYTNA STLEHHHHHH; SEQ ID NO: 43).

Protein SELEX was progressed in 3 rounds for each protein according to the scheme of the following Tables 3 to 5:

**Table 3**

| | ssDNA (DNA aptamer) | | TGFBRII | | Molar Ratio |
|---|---|---|---|---|---|
| Round | pM | *µ*g | pM | *µ*g | ssDNA : protein |
| round 1 | 500 | 13.95 | 50 | 1.75 | 10 : 1 |
| round 2 | 100 | 2.8 | 10 | 0.35 | 10 : 1 |
| round 3 | 100 | 2.8 | 5 | 0.18 | 20 : 1 |

**Table 4**

| | ssDNA (DNA aptamer) | | VEGF | | Molar Ratio |
|---|---|---|---|---|---|
| Round | pM | *µ*g | pM | *µ*g | ssDNA : protein |
| round 1 | 500 | 13.95 | 50 | 1.1 | 10 : 1 |
| round 2 | 100 | 2.8 | 10 | 0.22 | 10 : 1 |
| round 3 | 100 | 2.8 | 5 | 0.11 | 20 : 1 |

**Table 5**

| | ssDNA (DNA aptamer) | | CBF1 | | Molar Ratio |
|---|---|---|---|---|---|
| Round | pM | *µ*g | pM | *µ*g | ssDNA : protein |
| round 1 | 500 | 13.95 | 50 | 2.7 | 10 : 1 |
| round 2 | 100 | 2.8 | 10 | 0.54 | 10 : 1 |
| round 3 | 100 | 2.8 | 5 | 0.27 | 20 : 1 |

Ni-NTA magnetic bead 20ul of Qiagen Co., Ltd. was transferred to an E-tube, and the supernatant was removed using a magnetic rack. Using SELEX buffer (P B/W buffer) 100ul, beads were washed three times. The composition of SELEX buffer used for each protein herein was as follows: TGFBRII SELEX buffer (P B/W buffer): 12.5mM Tris-HCl (pH 7.5), 125mM NaCl, 1mM KCl, 1mM MgCl2, 1mM PMSF (phenylmethanesulfonylfluoride or phenylmethylsulfonyl fluoride);
VEGF SELEX buffer (P B/W buffer): 12.5mM Tris-HCl (pH 7.5), 125mM NaCl, 0.25mM DTT (dithiothreitol), 50mM KCl,
CBF1 SELEX buffer (P B/W buffer): 12.5mM Tris-HCl (pH 7.5), 125mM NaCl, 50mM KCl, 1mM DTT (dithiothreitol).

Then, the protein prepared in advance (TGFBRII ectodomain, VEGF, or CBF1) was added in an amount of 50pM to the SELEX buffer 100ul, and then it was under binding to beads at 4°C for 1 hour. After completing protein binding, the supernatant was removed, and beads were washed three times using SELEX buffer 100ul. Then, after adding SELEX buffer 100ul and biotinylated-ssDNA library 500pM prepared in Example 1 to the protein-bound Ni-NTA beads, it was reacted at 37°C for 1 hour, to bind DNA library (Example 1; HFL library and Random library) to protein-beads (DNA:protein=10:1; molar ratio). After that, the beads were washed with SELEX buffer 100ul three times, and then ssDNA elution buffer (50mM NaOH) 20ul was added, and it was heated at 95°C for 10 minutes, and ssDNA (single-stranded DNA) bound to the protein bound to beads was eluted. For the eluted ssDNA, symmetric PCR was performed under the following conditions, and thereby selected ssDNA was amplified:
(1) PCR mixture: Reverse primer (20pM), biotinylated-forward primer (20pM), dNTP (each InM), pfu(+) buffer (CANCERROP Co., Ltd.), pfu(+) DNA polymerase (1U; CANCERROP Co., Ltd.), template-ssDNA (20ng) prepared in Example 1;
(2) cycles: 95°C for 5min, start cycle (15 cycles) - 95°C for 30sec, 53°C for 30sec, 72°C for 10sec, end cycle - 72°C for 5min, 4°C hold.
   - Forward primer: 5'-biotin-TAG GGA AGA GAA GGA CAT ATG AT 3' (23mer)
   - Reverse primer: 5'-TCA AGT GGT CAT GTA CTA GTC AA-3' (23mer).

The dsDNA produced after performing PCR as above was used to obtain biotinylated-ssDNA (biotinylated-ssDNA for progressing SELEX 1 round: 86mer biotinylated-ssDNA prepared by commissioning to Bioneer Co., Ltd. described in Example 1; then, biotinylated-ssDNA for progressing SELEX 2 round and 3 round: biotinylated-ssDNA obtained by the process described below (method using streptavidin beads).

For the obtained PCR products, using streptavidin magnetic beads (NEB Co., Ltd.), biotinylated ssDNA was isolated.

More specifically, PCR product 10ug obtained by the method and D.W, 200mM NaOH, and 4x Wash/Binding Buffer (W/B buffer; 0.5 M NaCl, 20mM Tris-HCl (pH 7.5), 1mM EDTA (Ethylenediaminetetraacetic acid)) were mixed at a volume ratio of 1:1:1:1, and it was left at 37°C for 10 minutes, to prepare PCR product solution (Final concentration: 50mM NaOH, 1x wash/binding buffer). On the other hand, streptavidin magnetic bead (NEB Co., Ltd.) solution 250ul (pure bead amount: 1mg) was taken to remove the supernatant, and it was washed with 1x W/B buffer 200ul three times. The streptavidin magnetic bead in which the supernatant was removed was mixed well with the prepared PCR product (dsDNA state) solution 10ug, and it was reacted at 37°C for 1 hour to allow biotinylated-ssDNA to bind. After downing the streptavidin magnetic beads using a magnetic rack and then removing the supernatant, the beads were washed with 1x W/B buffer 200ul three times. After putting D.W 200ul to the biotinylated-ssDNA-bead obtained like this, it was left at 95°C for 20 minutes to elute biotinylated-ssDNA. The ssDNA obtained like this was used for the next SELEX round.

### Example 4. Ka value measurement through Indirect ELISA

The degree of binding (Ka value) between protein and the aptamer obtained for each protein by performing SELEX 3 round (See Example 3) by using the HFL or Random library prepared in Example 1 was measured by performing Indirect ELISA as follows.

Coating buffer (100mM sodium carbonate/bicarbonate (pH 9.6), 3.03g Na2CO3, 6.0g NaHCO3, 1000ml distilled water) 100ul and each protein 25pM/well were loaded to a plate (Pierce 96-Well Polystyrene Plates, White Opaque), and it was incubated at a room temperature for 2 hours. Then, after removing the buffer in the plate by vigorously spraying it on the sink, 200ul of wash buffer (PBS) was added to each well and it was washed 3 times repeatedly. Blocking buffer (5%(v/v) BSA in PBS) of 200ul was added to each well and it was incubated at a room temperature for 3 hours. Then, after removing the blocking buffer in the plate by vigorously spraying it on the sink, 200ul of wash buffer (PBS) was added to each well and it was washed three times.

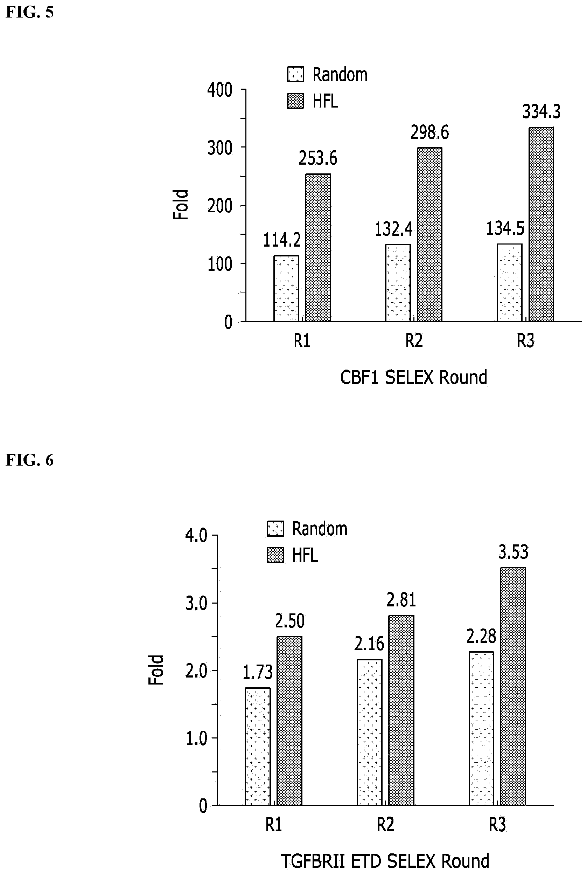

The biotinylated-ssDNA 0.2pM selected per SELEX round in Example 3 was loaded with 100 ul of the SELEX buffer of each target protein (See Example 3) into each well of the prepared plate in which each protein was coated, and it was incubated at 37°C for 1 hour (in the present example, 3 rounds were performed in total). After removing the solution in the plate by vigorously spraying it on the sink, 200ul of wash buffer (PBS) was added to each well, and it was washed three times repeatedly. The streptavidin-HRP protein (abcam) was diluted in blocking buffer (5%(v/v) BSA in PBS) to 10000:1 (v:v), and 200ul was loaded to each well, and then it was incubated at a room temperature under the darkroom condition for 2 hours. After removing the solution in the plate by vigorously spraying it on the sink, 200ul of wash buffer (PBS) was added to each well, and it was washed three times. After that, 100ul of ECL Substrate solution (SuperSignal ELISA Femto Substrate) was added to each well and the generated luminescence value was measured (measured by SPARK (TECAN)). For comparison, the luminescence value in case of no-treatment of the target protein was measured by the method the same as above and it was used as a background value. All tests were performed three times, and the average value was calculated and used for analysis of results. The result of dividing the obtained luminescence value by the background value was shown in FIG. 4 to FIG. 6.

As shown in FIG. 4 to FIG. 6, it could be confirmed that the aptamer obtained from the aptamer template having a stem-loop structure (HFL) had excellent binding ability to a target protein, even when performing a relatively less number of SELEX round (for example, 3 round), compared to the aptamer obtained from the conventional random library, and it could be confirmed that it showed high binding ability, for all the tested proteins having various pI values including protein having a low pI value with weak binding ability to the aptamer by electrostatic repulsive power (for example, TGFBRII ectodomain), compared to the aptamer obtained from the conventional random library.

## Claims

1. A nucleic acid structure represented by the following general formula 2:
5'-[(N)ₚ][(X)₁][G(N)ₙ][(X)ₘ][(N)_{q}]-3' (general formula 2)
wherein,
N is each independently selected from A, T or U, G, and C randomly, and
X is each independently selected from A, T or U, G, and C randomly, and the ratio of C or G is 55% or higher based on the total nucleotides of [(X)ₗ] or [(X)ₘ], and
[G(N)ₙ] is a site forming a loop structure, and n is the number of nucleotide N and an integer of 3 to 29, and
[(X)ₗ] and [(X)ₘ] are sites forming a stem structure by forming a base pair with antiparallel complementary sequences each other, and 1 and m are numbers of nucleotide X and each is an integer of 3 to 10, and
[(N)ₚ] and [(N)_{q}] are 5' end region and 3' end region, respectively, and p and q are numbers of nucleotide N, and each is independently an integer of 1 to 50, and p<q.

2. The nucleic acid structure according to claim 1,
wherein the [(X)ₗ] is 5'-GGAC-3', and [(X)ₘ] is 5'-GTCC-3' or 5'-GUCC-3' which is anti-parallel complementary to the [(X)i], and
the condition p<q is satisfied, and the p is selected from integers of 1 to 20, and the q is selected from integers of 15 to 50.

3. The nucleic acid structure according to claim 2, wherein the p is an integer of 1 to 10, and the q is an integer of 15 to 50.

4. The nucleic acid structure according to claim 1, wherein the nucleic acid structure is represented by the following general formula 3 or general formula 4:
NNNGGACGNN NNNNNGTCCN NNNNNNNNNN (general formula 3; SEQ ID NO: 44),
NNNGGACGNN NNNNNGUCCN NNNNNNNNNN (general formula 4; SEQ ID NO: 45),
wherein the N is each independently selected from A, T or U, G, and C.

5. An aptamer template comprising the nucleic acid structure of any one of claims 1 to 4.

6. A composition for preparing an aptamer comprising the nucleic acid structure of any one of claims 1 to 4.

7. A nucleic acid aptamer, prepared from the aptamer template of claim 5.

8. A method for preparing a target material-specific binding nucleic acid molecule, comprising
(1) contacting the nucleic acid structure of any one of claims 1 to 4 with a target material; and
(2) measuring whether to bind between the nucleic acid structure and target material.

9. The method for preparing a target material-specific binding nucleic acid molecule according to claim 8, further comprising
(3) determining the nucleic acid structure as a nucleic acid molecule specifically binding to the target material, when binding between the nucleic acid structure and target material is confirmed.

10. The method for preparing a target material-specific binding nucleic acid molecule according to claim 8, wherein the nucleic acid structure is two or more kinds having different sequences each other.

11. The method for preparing a target material-specific binding nucleic acid molecule according to claim 10, wherein the target material-specific binding nucleic acid molecule is a target material-specific nucleic acid aptamer.

12. A method for preparing a nucleic acid aptamer specifically binding to a target material, comprising
(i) contacting a library comprising one or more kinds of the nucleic acid structures of any one of claims 1 to 4 with beads in which a target material is immobilized;
(ii) washing the beads of the step (i); and
(iii) confirming the nucleic acid structure bound to the beads of the step (ii).

13. The method for preparing a nucleic acid aptamer according to claim 12, further comprising
(3) determining the nucleic acid structure as a nucleic acid aptamer specifically binding to the target material, when binding between the nucleic acid structure and target material is confirmed.

14. The method for preparing a nucleic acid aptamer according to claim 12, wherein the nucleic acid structure is two or more kinds having different sequences each other.

15. The method for preparing a nucleic acid aptamer according to claim 13, wherein the nucleic acid structure is two or more kinds having different sequences each other.
